# EUROPEAN PATENT APPLICATION

(11) **EP 3 141 178 A1**
(43) Date of publication of application: **15.03.2017**
(21) Application number: 15862891.7
(22) Date of filing: 14.10.2015
(51) Int. Cl.: A61B 1/00, A61B 1/04, G02B 23/24, H04N 7/18

(54) **DIAGNOSIS ASSISTANCE DEVICE AND DIAGNOSIS ASSISTANCE INFORMATION DISPLAY METHOD**

(30) Priority: 26.11.2014 JP 2014239157
(71) Applicant: Olympus Corporation, Tokyo 192-8507 (JP)
(72) Inventor: UCHIYAMA, Hiroki, Hachioji-shi Tokyo 192-8507 (JP); WATANABE, Toshiaki, Hachioji-shi Tokyo 192-8507 (JP); TAKEUCHI, Yuichi, Hachioji-shi Tokyo 192-8507 (JP)
(74) Representative: Gunzelmann, Rainer
(86) International application number: PCT/JP2015/078992
(87) International publication number: WO 2016/084504

(57) **Abstract**

A diagnosis assisting apparatus includes a region extraction section configured to perform a process for extracting, from among fluorescence images obtained by picking up images of fluorescence generated when a desired object in a subject is irradiated with excitation light, a reference region to be handled as a reference for a fluorescence generation state and a region of interest to be handled as a comparison target of the fluorescence generation state, respectively, a calculation processing section configured to perform a calculation process for calculating a calculation value indicating an intensity ratio of the fluorescence of the region of interest to the reference region based on a luminance value of each pixel included in the reference region and a luminance value of each pixel included in the region of interest, a storage section configured to store the calculation value calculated by the calculation processing section, and an image processing section configured to perform a process for causing a display apparatus to display information indicating a variation over time of the calculation value stored in the storage section.

## Description

### Technical Field

The present invention relates to a diagnosis assisting apparatus and a diagnosis assisting information display method, and more particularly, to a diagnosis assisting apparatus and a diagnosis assisting information display method used for fluorescence observation.

### Background Art

In endoscope observation in a medical field, fluorescence observation is conventionally practiced which is an observation technique of, for example, diagnosing whether or not a lesioned region is included in a desired object based on a fluorescence generation state when the desired object is irradiated with excitation light for exciting a fluorescent agent administered into a living body. For example, Japanese Patent Application Laid-Open Publication No. 2008-154846 discloses a fluorescence endoscope available for fluorescence observation.

Here, in endoscope observation, it is generally assumed to be difficult to observe an object while keeping a distance between an object and an endoscope constant. For this reason, in fluorescence observation using the endoscope, an image obtained by picking up an image of fluorescence emitted from the object may latently include a variation in the intensity of fluorescent light corresponding to a variation in the distance between the object and the endoscope. Therefore, fluorescence observation using the endoscope involves a problem that it is difficult to appropriately evaluate a change over time in a state of generation of fluorescence from the object while excluding influences of a variation in the intensity of fluorescence corresponding to a variation in the distance between the object and the endoscope.

On the other hand, Japanese Patent Application Laid-Open Publication No. 2008-154846 does not particularly mention techniques capable of solving the aforementioned problem or the like. Therefore, according to the configuration disclosed in Japanese Patent Application Laid-Open Publication No. 2008-154846, due to the difficulty in appropriately evaluating a change over time in a state of generation of fluorescence from the object, there is a problem associated with the aforementioned problem that an excessive burden may be imposed on an operator who diagnoses the object through fluorescence observation using the endoscope.

The present invention has been implemented in view of the aforementioned circumstances and it is an object of the present invention to provide a diagnosis assisting apparatus and a diagnosis assisting information display method capable of reducing a burden on an operator who makes a diagnosis through fluorescence observation using an endoscope.

### Disclosure of Invention

### Means for Solving the Problem

A diagnosis assisting apparatus according to an aspect of the present invention includes a region extraction section configured to perform a process for extracting, from among fluorescence images obtained by picking up images of fluorescence generated when a desired object in a subject is irradiated with excitation light, a reference region to be handled as a reference for a fluorescence generation state and a region of interest to be handled as a comparison target of the fluorescence generation state, respectively, a calculation processing section configured to perform a calculation process for calculating a calculation value indicating an intensity ratio of the fluorescence of the region of interest to the reference region based on a luminance value of each pixel included in the reference region and a luminance value of each pixel included in the region of interest, a storage section configured to store the calculation value calculated by the calculation processing section, and an image processing section configured to perform a process for causing a display apparatus to display information indicating a variation over time of the calculation value stored in the storage section.

A diagnosis assisting information display method according to an aspect of the present invention includes a region extracting step in a region extraction section of performing a process for extracting, from among fluorescence images obtained by picking up images of fluorescence generated when a desired object in a subject is irradiated with excitation light, a reference region to be handled as a reference for a fluorescence generation state and a region of interest to be handled as a comparison target of the fluorescence generation state, respectively, a calculation processing step in a calculation processing section of performing a calculation process for calculating a calculation value indicating an intensity ratio of the fluorescence of the region of interest to the reference region based on a luminance value of each pixel included in the reference region and a luminance value of each pixel included in the region of interest and storing the calculation value in a storage section, and an image processing step in an image processing section of performing a process for causing a display apparatus to display information indicating a variation over time of the calculation value stored in the storage section.

### Brief Description of the Drawings

Fig. 1 is a diagram illustrating a configuration of main parts of an endoscope system including a diagnosis assisting apparatus according to an embodiment;
Fig. 2 is a diagram for describing an example of an internal configuration of the endoscope system in Fig. 1;
Fig. 3 is a diagram illustrating an example of a fluorescence image used for processing by the diagnosis assisting apparatus according to the embodiment;
Fig. 4 is a diagram illustrating an example where a reference region Ar, a region of interest Ai1 and a region of interest Ai2 are extracted from the fluorescence image in Fig. 3;
Fig. 5 is a diagram illustrating an example of a diagnosis assisting image generated by the diagnosis assisting apparatus according to the embodiment;
Fig. 6 is a diagram illustrating an example of diagnosis assisting information generated by the diagnosis assisting apparatus according to the embodiment;
Fig. 7 is a diagram illustrating an example of a configuration of a fluorescence member used together with the diagnosis assisting apparatus according to the embodiment; and
Fig. 8 is a diagram illustrating an example of a fluorescence image picked up when the fluorescence member in Fig. 7 is disposed.

### Best Mode for Carrying Out the Invention

Hereinafter, an embodiment of the present invention will be described with reference to the accompanying drawings.

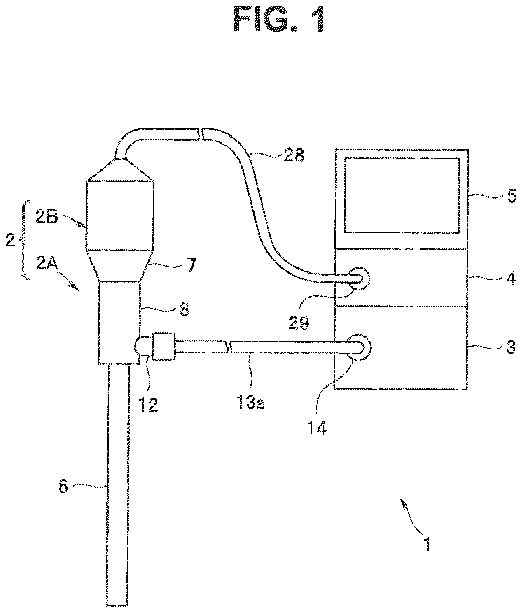

Fig. 1 to Fig. 8 relate to an embodiment of the present invention.

As shown in Fig. 1, an endoscope system 1 includes an endoscope 2 configured to be inserted into a subject and pick up an image of an object in the subject such as a living tissue and output the image as an image pickup signal, a light source apparatus 3 configured to supply illumination light for illuminating the object to the endoscope 2, a video processor 4 configured to apply signal processing to the image pickup signal outputted from the endoscope 2 to thereby generate and output an observation image or the like, and a monitor 5 configured to display the observation image or the like outputted from the video processor 4 on a screen. Fig. 1 is a diagram illustrating a configuration of main parts of the endoscope system including a diagnosis assisting apparatus according to the embodiment.

The endoscope 2 is constructed of an optical visual tube 2A provided with an elongated insertion portion 6 and a camera unit 2B attachable/detachable to/from an eyepiece part 7 of the optical visual tube 2A.

The optical visual tube 2A is constructed of the elongated insertion portion 6 inserted into the subject, a grasping portion 8 provided at a proximal end portion of the insertion portion 6, and an eyepiece part 7 provided at a proximal end portion of the grasping portion 8.

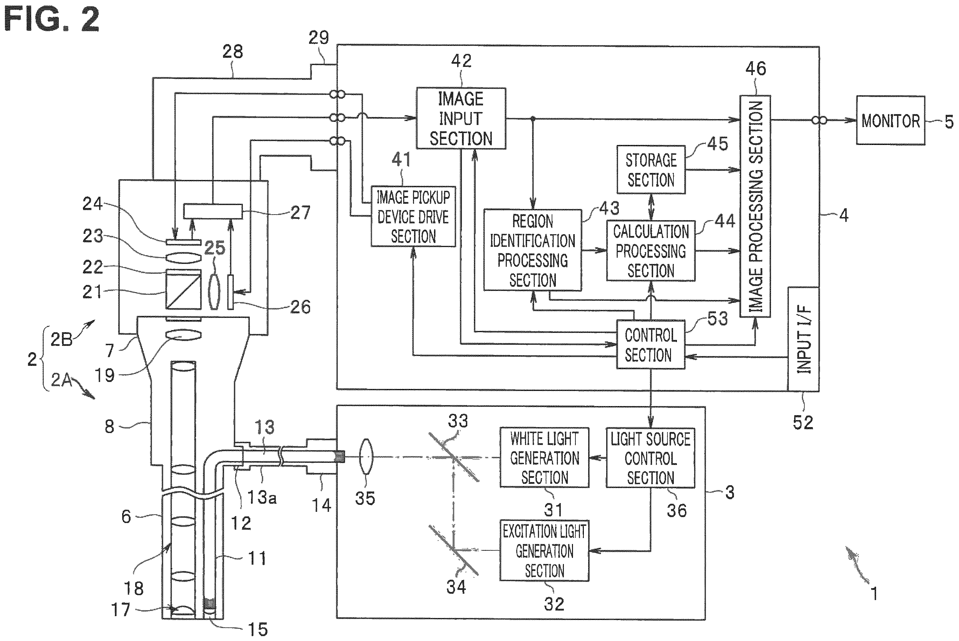

As shown in Fig. 2, a light guide 11 configured to transmit illumination light supplied via a cable 13a is inserted through the insertion portion 6. Fig. 2 is a diagram for describing an example of an internal configuration of the endoscope system in Fig. 1.

As shown in Fig. 2, an emission end portion of the light guide 11 is disposed in the vicinity of an illumination lens 15 at a distal end portion of the insertion portion 6. An incident end portion of the light guide 11 is disposed at a light guide pipe sleeve 12 provided in the grasping portion 8.

As shown in Fig. 2, a light guide 13 for transmitting illumination light supplied from the light source apparatus 3 is inserted into the cable 13a. Furthermore, a connection member (not shown) attachable/detachable to/from the light guide pipe sleeve 12 is provided at one end portion of the cable 13a. A light guide connector 14 attachable/detachable to/from the light source apparatus 3 is provided at the other end portion of the cable 13a.

An illumination window (not shown) provided with the illumination lens 15 for emitting illumination light transmitted by the light guide 11 to outside and an objective window (not shown) provided with an objective lens 17 for obtaining an optical image corresponding to light incident from outside are provided adjacent to each other on a distal end face of the insertion portion 6.

As shown in Fig. 2, a relay lens 18 for transmitting an optical image obtained by the objective lens 17 to the eyepiece part 7 is provided inside the insertion portion 6.

As shown in Fig. 2, an eyepiece lens 19 configured to allow an optical image transmitted by the relay lens 18 to be observed by naked eye is provided inside the eyepiece part 7.

The camera unit 2B is provided with a fluorescence image pickup system configured to pick up an image of fluorescence as return light incident via the eyepiece lens 19 in a fluorescence observation mode and generate a fluorescence image, and a white light image pickup system configured to pick up an image of reflected light of white light as return light incident via the eyepiece lens 19 in a white light observation mode and generate a white light image. The fluorescence image pickup system and the white light image pickup system are divided into two optical axes orthogonal to each other by a dichroic prism 21 having a spectral characteristic that reflects white light and transmits fluorescence. The camera unit 2B is configured to include a signal cable 28 provided with a signal connector 29 attachable/detachable to/from the video processor 4 at an end portion.

The fluorescence image pickup system of the camera unit 2B is provided with an excitation light cut filter 22 configured to have a spectral characteristic so as to cut a wavelength band EW of excitation light emitted from the light source apparatus 3, an image forming optical system 23 configured to form an image of fluorescence that passes through the dichroic prism 21 and the excitation light cut filter 22 and an image pickup device 24 configured to pick up an image of fluorescence formed by the image forming optical system 23.

The image pickup device 24 is constructed of, for example, a high sensitivity monochrome CCD. The image pickup device 24 is configured to perform image pickup operation corresponding to an image pickup device drive signal outputted from the video processor 4. The image pickup device 24 is configured to pick up an image of fluorescence formed by the image forming optical system 23 and generate and output a fluorescence image corresponding to the imaged fluorescence.

The white light image pickup system of the camera unit 2B is provided with an image forming optical system 25 configured to form an image of white light reflected by the dichroic prism 21 and an image pickup device 26 configured to pick up an image of white light, an image of which is formed by the image forming optical system 25.

The image pickup device 26 is constructed of a color CCD for which a primary color based or a complementary color based color filter is provided on an image pickup surface. The image pickup device 26 is also configured to perform image pickup operation corresponding to an image pickup device drive signal outputted from the video processor 4. The image pickup device 26 is also configured to pick up an image of white light, an image of which is formed by the image forming optical system 25 and generate and output a white light image corresponding to the imaged white light.

On the other hand, the camera unit 2B is provided with a signal processing circuit 27 configured to apply predetermined signal processing (correlation double sampling processing, gain adjustment processing, A/D conversion processing and the like) to the fluorescence image outputted from the image pickup device 24 and the white light image outputted from the image pickup device 26 and output the fluorescence image and the white light image subjected to the predetermined signal processing to the video processor 4 to which the signal cable 28 is connected.

The light source apparatus 3 is constructed of a white light generation section 31, an excitation light generation section 32, dichroic mirrors 33 and 34, a condensing lens 35 and a light source control section 36.

The white light generation section 31 is constructed of, for example, a lamp or an LED configured to emit wideband white light. The white light generation section 31 is configured to switch between a lighting state and a non-lighting state under the control of the light source control section 36. The white light generation section 31 is configured to generate white light having a light quantity corresponding to the control of the light source control section 36.

The excitation light generation section 32 is provided with an LED or the like configured to emit light (excitation light) of a predetermined wavelength band including an excitation wavelength of a fluorescent agent administered into a subject. The excitation light generation section 32 is configured to switch between a lighting state and a light-off state under the control of the light source control section 36. The excitation light generation section 32 is also configured to generate excitation light having a light quantity corresponding to the control of the light source control section 36.

The dichroic mirror 33 is formed so as to have an optical characteristic configured to transmit white light emitted from the white light generation section 31 to the condensing lens 35 side and reflect excitation light emitted from the excitation light generation section 32 to the condensing lens 35 side, for example.

The dichroic mirror 34 is formed so as to have an optical characteristic configured to reflect the excitation light emitted from the excitation light generation section 32 to the dichroic mirror 33 side, for example.

The condensing lens 35 is configured to condense the light incident via the dichroic mirror 33 so as to be emitted to the light guide 13.

The light source control section 36 is configured to perform control on the white light generation section 31 and the excitation light generation section 32 according to an illumination control signal outputted from the video processor 4.

The video processor 4 is constructed of an image pickup device drive section 41, an image input section 42, a region identification processing section 43, a calculation processing section 44, a storage section 45, an image processing section 46, an input I/F (interface) 52, and a control section 53.

The image pickup device drive section 41 is provided with, for example, a driver circuit. The image pickup device drive section 41 is also configured to generate and output an image pickup device drive signal under the control of the control section 53.

The image input section 42 is provided with, for example, a buffer memory and is configured to store images for one frame sequentially outputted from the signal processing circuit 27 of the camera unit 2B and output the stored images frame by frame to the control section 53. The image input section 42 is configured to output white light images stored in a white light observation mode frame by frame to the image processing section 46 under the control of the control section 53. The image input section 42 is configured to output fluorescence images stored in a fluorescence observation mode to the region identification processing section 43 and the image processing section 46 frame by frame under the control of the control section 53.

The region identification processing section 43 is configured to apply a labeling process to fluorescence images sequentially outputted frame by frame from the image input section 42 under the control of the control section 53, make a reference region Ar (which will be described later) and a region of interest Ai (which will be described later) included in the fluorescence images identifiable and output the fluorescence images subjected to the labeling process to the calculation processing section 44.

The calculation processing section 44 is provided with, for example, a calculation processing circuit. The calculation processing section 44 is configured to perform a calculation process for calculating a calculation value indicating an intensity ratio of fluorescence of the region of interest Ai to the reference region Ar based on a luminance value of each pixel included in the reference region Ar of fluorescence images sequentially outputted frame by frame from the region identification processing section 43 and a luminance value of each pixel included in the region of interest Ai of the fluorescence images under the control of the control section 53. The calculation processing section 44 is configured to output the calculation value obtained as a processing result of the aforementioned calculation process to the storage section 45 and/or the image processing section 46 under the control of the control section 53.

The storage section 45 is provided with, for example, a memory and is configured to assign a time stamp to the calculation value outputted from the calculation processing section 44 and store the calculation value.

The image processing section 46 is provided with an image processing circuit or the like to perform predetermined image processing. The image processing section 46 is configured to apply predetermined image processing to the white light images sequentially outputted frame by frame from the image input section 42 in the white light observation mode under the control of the control section 53, thereby generate a white light observation image and output the generated white light observation image to the monitor 5. The image processing section 46 is configured to apply predetermined image processing to the fluorescence images sequentially outputted frame by frame from the image input section 42 in the fluorescence observation mode under the control of the control section 53, thereby generate a fluorescence observation image and output the generated fluorescence observation image to the monitor 5.

On the other hand, the image processing section 46 is configured to perform a process for causing the monitor 5 to display diagnosis assisting information (which will be described later) based on the fluorescence image outputted from the region identification processing section 43, the calculation value outputted from the calculation processing section 44 and the calculation value read from the storage section 45 in the fluorescence observation mode under the control of the control section 53.

The input I/F 52 is provided with one or more input apparatuses that can give an instruction corresponding to a user's operation. More specifically, the input I/F 52 is provided with an observation mode changeover switch (not shown) configured to be able to give an instruction for setting (switching) the observation mode of the endoscope system 1 to either the white light observation mode or the fluorescence observation mode according to the user's operation, for example. The input I/F 52 is provided with a diagnosis assisting information display switch (not shown) configured to be able to set (switch) a display of diagnosis assisting information in the fluorescence observation mode to either ON or OFF according to the user's operation, for example. The input I/F 52 is constructed of a pointing device (not shown) capable of giving an instruction for setting each of the reference region Ar and the region of interest Ai within the fluorescence observation image displayed on the monitor 5 in the fluorescence observation mode according to the user's operation.

The control section 53 is provided with, for example, a CPU and is configured to generate an illumination control signal to emit illumination light corresponding to the observation mode of the endoscope system 1 based on the instruction issued by the observation mode changeover switch of the input I/F 52 and output the illumination control signal to the light source control section 36. The control section 53 is configured to control each of the image pickup device drive section 41, the image input section 42 and the image processing section 46 so as to perform operation corresponding to the observation mode of the endoscope system 1 based on the instruction issued by the observation mode changeover switch of the input I/F 52.

On the other hand, when the observation mode of the endoscope system 1 is set to the fluorescence observation mode and the display of diagnosis assisting information is set to ON, the control section 53 is configured to control the region identification processing section 43, the calculation processing section 44 and the image processing section 46 so as to extract each of the reference region Ar and the region of interest Ai from among the fluorescence images outputted from the image input section 42 based on an instruction issued by the pointing device of the input I/F 52 and display the diagnosis assisting information which is information that visualizes a fluorescence generation state of the region of interest Ai with respect to the extracted reference region Ar on the monitor 5.

Next, operation or the like of the endoscope system 1 of the present embodiment will be described.

First, the user such as an operator connects the respective sections of the endoscope system 1, turns on the power, and then operates the input I/F 52 and thereby gives an instruction for setting the observation mode of the endoscope system 1 to the white light observation mode.

Upon detecting that the white light observation mode is set, the control section 53 generates an illumination control signal for emitting white light from the light source apparatus 3 and outputs the illumination control signal to the light source control section 36. Upon detecting that the white light observation mode is set, the control section 53 controls the image pickup device drive section 41 so as to drive the image pickup device 26 of the camera unit 2B and stop driving of the image pickup device 24 of the camera unit 2B.

The light source control section 36 performs control to set the white light generation section 31 to a lighting state and set the excitation light generation section 32 to a non-lighting state in accordance with the illumination control signal outputted from the control section 53.

The image pickup device drive section 41 generates an image pickup device drive signal to stop image pickup operation under the control of the control section 53 and outputs the image pickup device drive signal to the image pickup device 24, and generates an image pickup device drive signal to perform image pickup operation during a predetermined exposure period EA and a predetermined reading period RA and outputs the image pickup device drive signal to the image pickup device 26.

When the light source control section 36 and the image pickup device drive section 41 perform the above-described operations, an object is irradiated with white light as illumination light, an image of reflected light of the white light is picked up by the image pickup device 26 and a white light image obtained by picking up an image of the reflected light of the white light is outputted to the image input section 42 via the signal processing circuit 27.

Upon detecting that the white light observation mode is set, the control section 53 controls the image input section 42 so as to output white light images sequentially outputted from the camera unit 2B frame by frame to the image processing section 46. Furthermore, upon detecting that the white light observation mode is set, the control section 53 controls the image processing section 46 so as to perform a predetermined image process on the white light images sequentially outputted from the image input section 42 frame by frame.

When the control section 53 performs the above-described control, the white light observation image is displayed on the monitor 5.

On the other hand, the user inserts the insertion portion 6 into the subject while watching the white light observation image displayed on the monitor 5, and thereby disposes the distal end portion of the insertion portion 6 in the vicinity of a desired object. After disposing the distal end portion of the insertion portion 6 in the vicinity of the desired object, the user operates the input I/F 52 to give an instruction for setting the observation mode of the endoscope system 1 to the fluorescence observation mode.

Here, more specific operation or the like carried out when the observation mode of the endoscope system 1 is set to the fluorescence observation mode will be described. Note that a case where the setting of a display of diagnosis assisting information is switched from OFF to ON will be described as an example below. The description hereinafter assumes that before the observation mode of the endoscope system 1 is set to the fluorescence observation mode, a fluorescent agent that emits fluorescence corresponding to the excitation light emitted from the excitation light generation section 32 has been administered into the subject in advance.

Upon detecting that the fluorescence observation mode is set, the control section 53 generates an illumination control signal for causing the light source apparatus 3 to emit excitation light and outputs the illumination control signal to the light source control section 36. Moreover, upon detecting that the fluorescence observation mode is set, the control section 53 controls the image pickup device drive section 41 so as to drive the image pickup device 24 of the camera unit 2B and stop driving the image pickup device 26 of the camera unit 2B.

In response to the illumination control signal outputted from the control section 53, the light source control section 36 performs control to set the white light generation section 31 to a non-lighting state and set the excitation light generation section 32 to a lighting state.

Under the control of the control section 53, the image pickup device drive section 41 generates an image pickup device drive signal to stop image pickup operation, outputs the image pickup device drive signal to the image pickup device 26, generates an image pickup device drive signal to cause the image pickup device 26 to perform image pickup operation for a predetermined exposure period EB and for a predetermined reading period RB, and outputs the image pickup device drive signal to the image pickup device 24.

When the light source control section 36 and the image pickup device drive section 41 perform the above-described operations, a desired object is irradiated with excitation light as illumination light, an image of fluorescence emitted from the fluorescent agent excited by the excitation light is picked up by the image pickup device 24, a fluorescence image obtained by image pickup performed on the fluorescence is outputted to the image input section 42 via the signal processing circuit 27.

Upon detecting that the fluorescence observation mode is set and the display of the diagnosis assisting information is set to OFF, the control section 53 controls the image input section 42 so as to output fluorescence images sequentially outputted from the camera unit 2B to the image processing section 46 frame by frame. Upon detecting that the fluorescence observation mode is set and the display of the diagnosis assisting information is set to OFF, the control section 53 controls the image processing section 46 so as to apply a predetermined image process to fluorescence images sequentially outputted from the image input section 42 frame by frame.

When the control section 53 performs the above-described control, the fluorescence observation image is displayed on the monitor 5.

On the other hand, the user operates the input I/F 52 while watching the fluorescence observation image displayed on the monitor 5 and thereby gives an instruction for switching the setting of the display of the diagnosis assisting information from OFF to ON.

Upon detecting that the display of the diagnosis assisting information is set to ON, the control section 53 controls the image processing section 46 so as to display a character string or the like for urging settings of the reference region Ar and the region of interest Ai within the fluorescence observation image together with a fluorescence observation image.

By operating the input I/F 52 while watching the character string displayed on the monitor 5 together with the fluorescence observation image, the user gives instructions for setting one reference region Ar to be handled as a reference of the fluorescence generation state and one or more region of interests Ai to be handled as comparison targets of the fluorescence generation state respectively from the fluorescence generation region included in the fluorescence observation image. Note that the reference region Ar and the region of interest Ai of the present embodiment are assumed to be set as pixel regions provided with one or more pixels.

The control section 53 provided with a function as a region extraction section performs processes for extracting the reference region Ar and the region of interest Ai respectively from among fluorescence images outputted from the image input section 42 based on instructions issued by the input I/F 52.

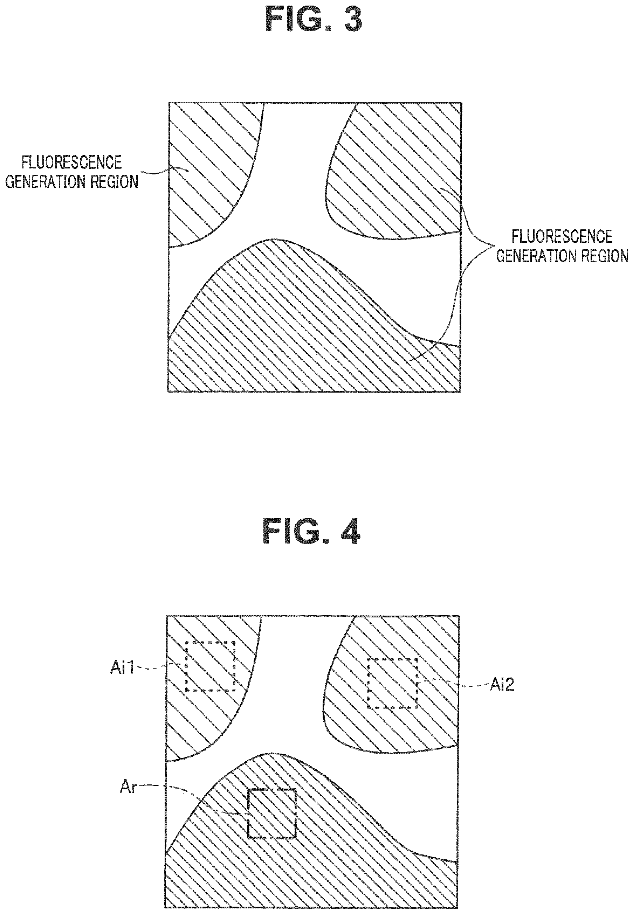

Hereinafter, specific processes or the like will be described in a case where one reference region Ar shown by a single-dot dashed line in Fig. 4 and two region of interests Ai1 and Ai2 shown by a broken line in Fig. 4 are respectively extracted from a fluorescence image including fluorescence generation regions shown by, for example, diagonal lines in Fig. 3. Fig. 3 is a diagram illustrating an example of a fluorescence image used for processing by the diagnosis assisting apparatus according to the embodiment. Fig. 4 is a diagram illustrating an example of a case where the reference region Ar, the region of interest Ai1 and the region of interest Ai2 are extracted from the fluorescence image in Fig. 3.

After extracting the reference region Ar, the region of interest Ai1 and the region of interest Ai2 from fluorescence images outputted from the image input section 42, the control section 53 controls the region identification processing section 43, the calculation processing section 44 and the image processing section 46 so as to display information on an intensity ratio of current fluorescence of the region of interest Ai1 to the reference region Ar and information on an intensity ratio of current fluorescence of the region of interest Ai2 to the reference region Ar on the monitor 5 as diagnosis assisting information.

Under the control of the control section 53, the region identification processing section 43 applies a labeling process to fluorescence images sequentially outputted frame by frame from the image input section 42, thereby makes the reference region Ar, the region of interests Ai1 and Ai2 included in the fluorescence images identifiable respectively and outputs the fluorescence images subjected to the labeling process to the calculation processing section 44.

Under the control of the control section 53, the calculation processing section 44 performs a calculation process for acquiring an average value or a maximum value of a luminance value of each pixel of the reference region Ar included in fluorescence images sequentially outputted from the region identification processing section 43 frame by frame as a representative value RV, calculating a calculation value AV1 which is a value of a ratio obtained by dividing the luminance value of the region of interest Ai1 included in the fluorescence image by the representative value RV for each pixel of the region of interest Ai1 and calculating a calculation value AV2 which is a value of a ratio obtained by dividing the luminance value of the region of interest Ai2 included in the fluorescence image by the representative value RV for each pixel of the region of interest Ai2. Under the control of the control section 53, the calculation processing section 44 outputs the calculation values AV1 and AV2 of each pixel obtained as the processing result of the aforementioned calculation process to the storage section 45 and the image processing section 46 respectively.

Under the control of the control section 53, the image processing section 46 performs a process for acquiring color information corresponding to each pixel of the region of interests Ai1 and Ai2 included in fluorescence images sequentially outputted frame by frame from the region identification processing section 43 from among a plurality of pieces of color information predetermined based on the magnitudes of the calculation values AV1 and AV2 outputted from the calculation processing section 44 and performs a process for outputting diagnosis assisting images which are colored region of interests Ai1 and Ai2 of the fluorescence images using the acquired color information to the monitor 5.

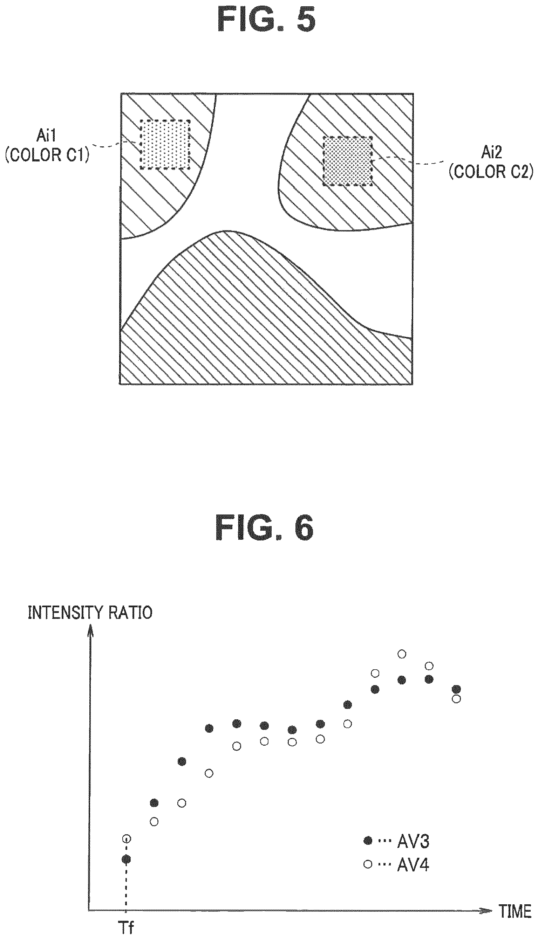

According to the aforementioned processes of the image processing section 46, for example, when the calculation values AV1 calculated at the respective pixels of the region of interest Ai1 in Fig. 4 are identical values, the calculation values AV2 calculated at the respective pixels of the region of interest Ai2 in Fig. 4 are identical values, and the calculation value AV1 and the calculation value AV2 are different values, a diagnosis assisting image shown in Fig. 5 can be displayed on the monitor 5. Fig. 5 is a diagram illustrating an example of the diagnosis assisting image generated by the diagnosis assisting apparatus according to the embodiment.

According to the diagnosis assisting image in Fig. 5, for example, information indicating the intensity ratio of the current fluorescence of the region of interest Ai1 to the reference region Ar is displayed on the monitor 5 with a color C1 and information indicating the intensity ratio of the current fluorescence of the region of interest Ai2 to the reference region Ar is displayed on the monitor 5 with a color C2. That is, the diagnosis assisting image in Fig. 5 includes, as diagnosis assisting information, the color C1 which is color information that visualizes the intensity ratio of the current fluorescence of the region of interest Ai1 to the reference region Ar and the color C2 which is color information that visualizes the intensity ratio of the current fluorescence of the region of interest Ai2 to the reference region Ar.

Note that the present embodiment is not limited to one that performs the aforementioned process, but may also be one that performs a process for displaying information indicating a variation over time of an intensity ratio of fluorescence as the diagnosis assisting information, as will be described later, for example. Note that specific description relating to parts to which existing operation or the like is applicable will be omitted hereinafter as appropriate for simplicity of description.

After extracting the reference region Ar, the region of interest Ai1 and the region of interest Ai2 from fluorescence images outputted from the image input section 42, the control section 53 controls the region identification processing section 43, the calculation processing section 44 and the image processing section 46 so as to display on the monitor 5, a variation over time of the intensity ratio of fluorescence of the region of interest Ai1 to the reference region Ar and a variation over time of the intensity ratio of fluorescence of the region of interest Ai2 to the reference region Ar as diagnosis assisting information.

Under the control of the control section 53, the region identification processing section 43 applies a labeling process to fluorescence images sequentially outputted from the image input section 42 frame by frame, thereby makes the reference region Ar, and the region of interests Ai1 and Ai2 identifiable respectively and outputs the fluorescence images subjected to the labeling process to the calculation processing section 44.

Under the control of the control section 53, the calculation processing section 44 performs a calculation process for acquiring an average value or a maximum value of a luminance value of each pixel of the reference region Ar included in fluorescence images sequentially outputted from the region identification processing section 43 frame by frame as a representative value RV, acquiring an average value or a maximum value of a luminance value of each pixel of the region of interest Ai1 included in fluorescence images as a representative value RV1, and acquiring an average value or a maximum value of a luminance value of each pixel of the region of interest Ai2 included in the fluorescence images as a representative value RV2 and further calculating a calculation value AV3 which is a value of a ratio obtained by dividing the representative value RV1 by the representative value RV (=RV1/RV) and a calculation value AV4 which is a value of a ratio obtained by dividing the representative value RV2 by the representative value RV (=RV2/RV). Under the control of the control section 53, the calculation processing section 44 simultaneously outputs the calculation values AV3 and AV4 obtained as the processing result of the aforementioned calculation process to the storage section 45.

The storage section 45 performs a process for assigning a time stamp indicating the same time to the calculation values AV3 and AV4 simultaneously inputted from the calculation processing section 44 and storing the calculation values AV3 and AV4.

That is, according to the aforementioned process, for example, the calculation values AV3 and AV4 obtained as the processing result of the calculation processing section 44 are stored in the storage section 45 frame by frame using the time Tf corresponding to a time immediately after setting the reference region Ar, the region of interest Ai1 and the region of interest Ai2 as a starting point.

Under the control of the control section 53, the image processing section 46 performs a process for reading the calculation values AV3 stored in time sequence in the storage section 45, plotting the read calculation values AV3 arranged in time sequence on a graph and outputting the graph to the monitor 5 as diagnosis assisting information. Under the control of the control section 53, the image processing section 46 performs a process for reading calculation values AV4 stored in time sequence in the storage section 45, plotting the read calculation values AV4 arranged in time sequence on a graph and outputting the graph to the monitor 5 as diagnosis assisting information.

According to the aforementioned process of the image processing section 46, it is possible to display diagnosis assisting information as shown, for example, in Fig. 6 on the monitor 5. Fig. 6 is a diagram illustrating an example of the diagnosis assisting information generated by the diagnosis assisting apparatus according to the embodiment.

According to the diagnosis assisting information in Fig. 6, variations over time in the calculation value AV3 using the time Tf as a starting point are displayed on the monitor 5 as a plurality of black points and variations over time in the calculation value AV4 using the time Tf as a starting point are displayed on the monitor 5 as a plurality of white points.

Note that the present embodiment is not limited to one in which variations over time in the calculation values AV3 and AV4 are displayed as diagnosis assisting information, but a rate of variations over time in the calculation values AV3 and AV4 may also be displayed as diagnosis assisting information, for example.

The present embodiment is not limited to one in which the aforementioned processes are performed, but may also be one in which a process is performed for displaying a value of an intensity ratio of fluorescence at a desired pixel position within the region of interest as the diagnosis assisting information as will be described below, for example.

After extracting the reference region Ar, the region of interest Ai1 and the region of interest Ai2 from fluorescence images outputted from the image input section 42, the control section 53 controls the image processing section 46 so as to display a character string that urges a selection of one pixel position from the extracted region of interest Ai1 and region of interest Ai2 or the like, together with the fluorescence observation image.

While watching the character string displayed on the monitor 5 together with the fluorescence observation image, the user operates the input I/F 52 and thereby gives an instruction for selecting one interested pixel PT from among the reference region Ar, the region of interest Ai1 and the region of interest Ai2.

Based on the instruction issued from the input I/F 52, the control section 53 specifies an interested pixel PT from among fluorescence images outputted from the image input section 42 and controls the region identification processing section 43, the calculation processing section 44 and the image processing section 46 so as to display on the monitor 5, a value of an intensity ratio of the current fluorescence of the interested pixel PT to the reference region Ar.

Under the control of the control section 53, the region identification processing section 43 applies a labeling process to fluorescence images sequentially outputted from the image input section 42 frame by frame, thereby makes the reference region Ar, the region of interests Ai1 and Ai2 included in the fluorescence image identifiable respectively and outputs the fluorescence images subjected to the labeling process to the calculation processing section 44.

Under the control of the control section 53, the calculation processing section 44 performs a calculation process for acquiring an average value or a maximum value of a luminance value of each pixel of the reference region Ar included in fluorescence images sequentially outputted from the region identification processing section 43 frame by frame as a representative value RV and further calculating a calculation value AV5 which is a value of a ratio obtained by dividing a luminance value PTB of the interested pixel PT by the representative value RV (=PTB/RV).

Under the control of the control section 53, the image processing section 46 performs a process for outputting the calculation value AV5 outputted from the calculation processing section 44 to the monitor 5 as diagnosis assisting information.

That is, according to the aforementioned process of the image processing section 46, the current calculation value AV5 at the interested pixel PT selected from the region of interests Ai1 and Ai2 is displayed on the monitor 5 as diagnosis assisting information.

The present embodiment is not limited to those which perform the above-described processes, and, for example, the embodiment may also perform a process for displaying on the monitor 5, a predetermined character string for indicating that the calculation value AV3 and/or the calculation value AV4 vary over time to reach a predetermined value TH1.

The present embodiment is not limited to those which perform the above-described processes, and, for example, the embodiment may also perform a process for displaying with blinking on the monitor 5, a pixel group in which the calculation value AV1 varies over time to reach a predetermined value TH2 and a pixel group in which the calculation value AV2 changes over time to reach the predetermined value TH2 among the respective pixels included in the region of interests Ai1 and Ai2.

The present embodiment is not limited to those which perform the above-described processes, and, for example, the embodiment may also perform a process for displaying on the monitor 5, a time period required from the time Tf to Tg when the calculation value AV3 and/or calculation value AV4 vary over time to reach a predetermined value TH3 at a time Tg.

On the other hand, according to the present embodiment, for example, in the fluorescence observation mode, the user may operate the input I/F 52 to give an instruction for setting a region where the reference fluorescent substance having a known fluorescence characteristic for excitation light emitted from the light source apparatus 3 is disposed as the reference region Ar. Note that in the case where the reference region Ar is set using such a method, for example, auxiliary calibration means for fluorescence observation or the like disclosed in Japanese Patent Application Laid-Open Publication No. 2005-300540 may be used as a reference fluorescent substance.

For example, in the fluorescence observation mode, the present embodiment may also be configured to perform such a process as to extract the region where the reference fluorescent substance is disposed as the reference region Ar based on a fluorescence image obtained by picking up an image of the reference fluorescent substance having a known fluorescence characteristic for excitation light emitted from the light source apparatus 3. Here, operation or the like of the endoscope system 1 when such a process is performed will be described below.

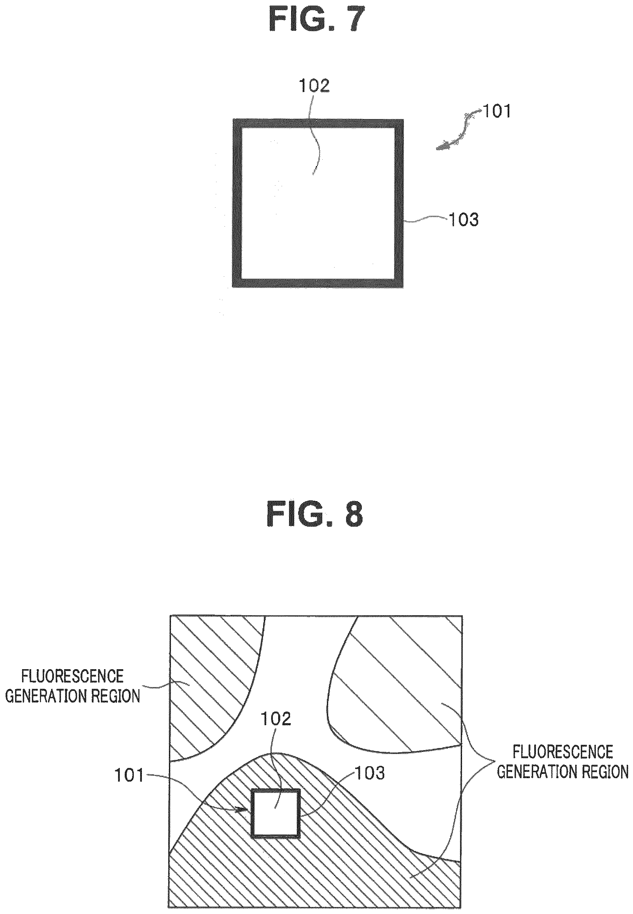

While watching the fluorescence observation image displayed on the monitor 5, the user disposes a fluorescence member 101 illustrated in Fig. 7 on the surface of a desired object. After disposing the fluorescence member 101 on the surface of the desired object, the user operates the input I/F 52 and gives an instruction for switching the setting of display of diagnosis assisting information from OFF to ON. Fig. 7 is a diagram illustrating an example of a configuration of the fluorescence member used together with the diagnosis assisting apparatus according to the embodiment.

The fluorescence member 101 is formed as a flat plate member having a square shape in a plan view as shown in, for example, Fig. 7. Note that the fluorescence member 101 may also be formed in a different shape in a plan view such as a star shape as long as it includes a straight line which is not existent in a living body.

On the other hand, the fluorescence member 101 is provided with a reference fluorescent substance 102 having a known fluorescence characteristic for excitation light emitted from the light source apparatus 3 and a frame member 103 provided so as to surround an outer edge portion of the reference fluorescent substance 102 as shown in Fig. 7.

The reference fluorescent substance 102 is formed by covering the surface of the fluorescent substance such as quantum dots with glass.

The frame member 103 is formed using a non-fluorescent member that generates no fluorescence corresponding to excitation light emitted from the light source apparatus 3 such as black PEEK (polyether ether ketone) resin.

That is, in the fluorescence observation mode, when the fluorescence member 101 is disposed on the surface of the desired object, an image of fluorescence is picked up by the image pickup device 24 in which a boundary between the reference fluorescent substance 102 and a fluorescence generation region other than the reference fluorescent substance 102 is emphasized by the frame member 103 as shown, for example, in Fig. 8 and outputted from the image input section 42. Fig. 8 is a diagram illustrating an example of the fluorescence image picked up when the fluorescence member in Fig. 7 is arranged.

Upon detecting that the display of diagnosis assisting information is set to ON, the control section 53 performs a process for specifying the inner region surrounded by the frame member 103 as a region where the reference fluorescent substance 102 exists based on a fluorescence image outputted from the image input section 42 and extracting the specified region as the reference region Ar.

More specifically, the control section 53 performs processes such as applying edge extraction to a fluorescence image outputted from the image input section 42 to thereby generate an edge image, applying Hough transform to the edge image generated to thereby extract a linear shape, specifying the inner region surrounded by the extracted linear shape as the region where the reference fluorescent substance 102 exists and extracting the specified region as the reference region Ar.

As described above, according to the present embodiment, the monitor 5 can display diagnosis assisting information that visualizes a fluorescence generation state of one or more region of interests Ai with respect to one reference region Ar. As a result, according to the present embodiment, it is possible to reduce a burden on an operator who makes a diagnosis based on fluorescence observation using an endoscope.

Note that the present embodiment may also be configured to display only one piece of diagnosis assisting information on the monitor 5 or display a plurality of pieces of diagnosis assisting information on the monitor 5 simultaneously. The present embodiment may also be configured to display on the monitor 5, diagnosis assisting information superimposed on the fluorescence observation image or display the diagnosis assisting information or diagnosis assisting image in a display region different from the display region of the fluorescence observation image on the monitor 5.

The present invention is not limited to the aforementioned embodiment, but it goes without saying that various modifications or applications can be made without departing from the spirit and scope of the present invention.

The present application claims a priority based on Japanese Patent Application No. 2014-239157, filed in Japan on November 26, 2014, the disclosure of which is incorporated in the specification, the claims and the drawings of the present application by reference.

## Claims

1. A diagnosis assisting apparatus comprising:
a region extraction section configured to perform a process for extracting, from among fluorescence images obtained by picking up images of fluorescence generated when a desired object in a subject is irradiated with excitation light, a reference region to be handled as a reference for a fluorescence generation state and a region of interest to be handled as a comparison target of the fluorescence generation state, respectively;
a calculation processing section configured to perform a calculation process for calculating a calculation value indicating an intensity ratio of the fluorescence of the region of interest to the reference region based on a luminance value of each pixel included in the reference region and a luminance value of each pixel included in the region of interest;
a storage section configured to store the calculation value calculated by the calculation processing section; and
an image processing section configured to perform a process for causing a display apparatus to display information indicating a variation over time of the calculation value stored in the storage section.

2. The diagnosis assisting apparatus according to claim 1, wherein the calculation processing section performs, as a calculation process for calculating the calculation value, a process for acquiring an average value or a maximum value of a luminance value of each pixel included in the reference region as a first representative value, acquiring an average value or a maximum value of a luminance value of each pixel included in the region of interest as a second representative value and calculating a value of a ratio obtained by dividing the second representative value by the first representative value.

3. The diagnosis assisting apparatus according to claim 1,
wherein the calculation processing section further performs a calculation process for acquiring an average value or a maximum value of a luminance value of each pixel included in the reference region as a representative value and calculating a value of a ratio obtained by dividing the luminance value of the region of interest by the representative value for each pixel of the region of interest, and
the image processing section further performs a process for causing the display apparatus to display color information corresponding to a magnitude of the value of the ratio calculated by the calculation processing section.

4. The diagnosis assisting apparatus according to claim 1,
wherein the calculation processing section further performs a calculation process for acquiring an average value or a maximum value of a luminance value of each pixel included in the reference region as a representative value and calculating a value of a ratio obtained by dividing the luminance value of one region of interest selected from among the respective pixels of the region of interest by the representative value, and
the image processing section further performs a process for causing the display apparatus to display the value of the ratio calculated by the calculation processing section.

5. The diagnosis assisting apparatus according to claim 1, wherein the region extraction section performs a process for extracting, from among the fluorescence images, a region where a reference fluorescent substance having a known fluorescence characteristic with respect to the excitation light exists as the reference region.

6. The diagnosis assisting apparatus according to claim 5, wherein when a frame member formed using a non-fluorescent member that does not produce the fluorescence in accordance with the excitation light is provided at an outer edge portion of the reference fluorescent substance, the region extraction section performs a process for specifying an inner region surrounded by the frame member as a region
where the reference fluorescent substance exists based on the fluorescence image and extracting the specified region as the reference region.

7. A diagnosis assisting information display method comprising:
a region extracting step in a region extraction section of performing a process for extracting, from among fluorescence images obtained by picking up images of fluorescence generated when a desired object in a subject is irradiated with excitation light, a reference region to be handled as a reference for a fluorescence generation state and a region of interest to be handled as a comparison target of the fluorescence generation state, respectively;
a calculation processing step in a calculation processing section of performing a calculation process for calculating a calculation value indicating an intensity ratio of the fluorescence of the region of interest to the reference region based on a luminance value of each pixel included in the reference region and a luminance value of each pixel included in the region of interest and causing a storage section to store the calculation value; and
an image processing step in an image processing section of performing a process for causing a display apparatus to display information indicating a variation over time of the calculation value stored in the storage section.

8. The diagnosis assisting information display method according to claim 7,
wherein in the calculation processing step, as a calculation process for calculating the calculation value, a process is performed for acquiring an average value or a maximum value of a luminance value of each pixel included in the reference region as a first representative value, acquiring an average value or a maximum value of a luminance value of each pixel included in the region of interest as a second representative value and calculating a value of a ratio obtained by dividing the second representative value by the first representative value.

9. The diagnosis assisting information display method according to claim 7,
wherein in the calculation processing step, a calculation process is further performed for acquiring an average value or a maximum value of a luminance value of each pixel included in the reference region as a representative value and calculating a value of a ratio obtained by dividing the luminance value of the region of interest by the representative value for each pixel of the region of interest, and
in the image processing step, a process is further performed for causing the display apparatus to display color information corresponding to the magnitude of the value of the ratio calculated in the calculation processing step.

10. The diagnosis assisting information display method according to claim 7,
wherein in the calculation processing step, a calculation process is further performed for acquiring an average value or a maximum value of a luminance value of each pixel included in the reference region as a representative value and calculating a value of a ratio obtained by dividing the luminance value of one region of interest selected from among the respective pixels of the region of interest by the representative value, and
in the image processing step, a process is further performed for causing the display apparatus to display the value of the ratio calculated in the calculation processing step.

11. The diagnosis assisting information display method according to claim 7,
wherein in the region extraction step, a process is performed for extracting a region where a reference fluorescent substance having a known fluorescence characteristic with respect to the excitation light exists as the reference region.

12. The diagnosis assisting information display method according to claim 11,
wherein when a frame member formed using a non-fluorescent member that does not produce the fluorescence in accordance with the excitation light is provided at an outer edge portion of the reference fluorescent substance, in the region extraction step, a process is performed for specifying an inner region surrounded by the frame member as a region where the reference fluorescent substance exists based on the fluorescence image and extracting the specified region as the reference region.
